# EUROPEAN PATENT SPECIFICATION

(11) **EP 0 431 879 B1**
(45) Date of publication and mention of the grant of the patent: **15.02.1995**
(21) Application number: 90313126.6
(22) Date of filing: 04.12.1990
(51) Int. Cl.: A61B 19/02

(54) **A secure container for disposable sharps**
Sicherheitsbehälter für einmal benutzbare spitze Instrumente
Conteneur de sécurité pour objets pointus jetables

(30) Priority: 05.12.1989 US 446121
(43) Date of publication of application: 12.06.1991
(73) Proprietor: MED-SAFE SYSTEMS, INC., Carlsbad, California 92009 (US)
(72) Inventor: Shillington, Richard, Leucadia, California 92024 (US)
(74) Representative: Spall, Christopher John

(56) References cited:
- WO-A-89/01905
- FR-A- 2 617 813
- GB-A- 2 087 360

## Description

### BACKGROUND OF THE INVENTION

The present invention relates to containers for disposable hospital sharps and waste, and pertains particularly to a secure disposable container assembly for disposition of hospital sharps, objects and wastes.

Hospitals and clinics use great quantities of sharps, such as needles, syringes, surgical blades, and the like, that are disposed of rather than cleaned and reused. It is necessary that the sharps be disposed of in a manner that prevents them being reused without sterilization. In particular, it is necessary to keep them from falling into the hands of those, such as drug users and the like, that are likely to use them without proper sterilization.

Numerous containers have been developed in recent years, which are reasonably secure and disposable for receiving and disposing of hospital sharps, wastes and the like. Many of these containers however do not provide adequate security against pilfering of used syringes and the like from such containers. While containers have been developed which cannot readily be reopened and articles cannot be easily removed therefrom, such containers must be kept in a secure place to prevent unauthorized removal.

It is essential that access openings into the containers be easy and convenient to use, yet prevent access thereto for removal of articles. This requires that the opening be adequate in size to receive the article, be conveniently located and that it has a suitable closure. While many excellent closures exist, improvements are desirable.

In prior U.S. Patent No. 4,702,385, of which I am co-inventor, we disclose a security mounting device for disposable containers. That device includes a metal mounting bracket, with a complex metal latching arrangement for securely latching a disposable container within a secured container housing. While that prior device is suitable for many applications, certain improvements are desirable.

In GB-A-2 087 360-A a disposable container for used items of medical equipment is disclosed which has a self-closing door flap which covers an access opening to the interior of the container. However, as soon as the closure is pivoted away from the opening, the opening is fully open for total access to the interior of the container.

In WIPO Patent Application No. WO 89/01905 a sharps disposal system is disclosed having a one-way valve in the form of a paddle wheel which rotates to drop the used sharps into a container. The container has a lid having an aperture with a hinged door which must be opened to receive the sharps.

It is, therefore, desirable that an improved securable disposable container assembly be provided.

### SUMMARY AND OBJECTS OF THE INVENTION

It is the primary object of the present invention to provide an improved securable disposable container assembly.

In accordance with a primary aspect of the present invention, a secure disposable container assembly comprises a housing for securely mounting to a support member, and having an access opening in a top front for receiving a disposable article and preventing access to the interior of the container by the human hand. Means are provided for locking the housing to a support bracket to prevent removal of the container.

Dependent claims 2-10 disclose further embodiments.

### BRIEF DESCRIPTION OF THE DRAWING

The above and other objects and advantages of the present invention will become apparent from the following description when read in conjunction with the accompanying drawings wherein:
Fig. 1 is a front elevation view of an exemplary embodiment of the invention;
Fig. 2 is a top plan view of the embodiment of Fig. 1;
Fig. 3 is a detailed partial view in section taken on line 3-3 of Fig. 2;
Fig. 4 is a detailed partial view in section taken on line 4-4 of Fig. 3;
Fig. 5 is a detailed partial back view of the container with portions broken away to reveal details;
Fig. 6 is a partial side elevation view in section;
Fig. 7 is a partial side elevation view in section showing the container mount;
Fig. 8 is a view taken generally on line 8-8 of Fig. 7; and,
Fig. 9 is a top plan view of the latch release key.

### DETAILED DESCRIPTION OF A PREFERRED EMBODIMENT

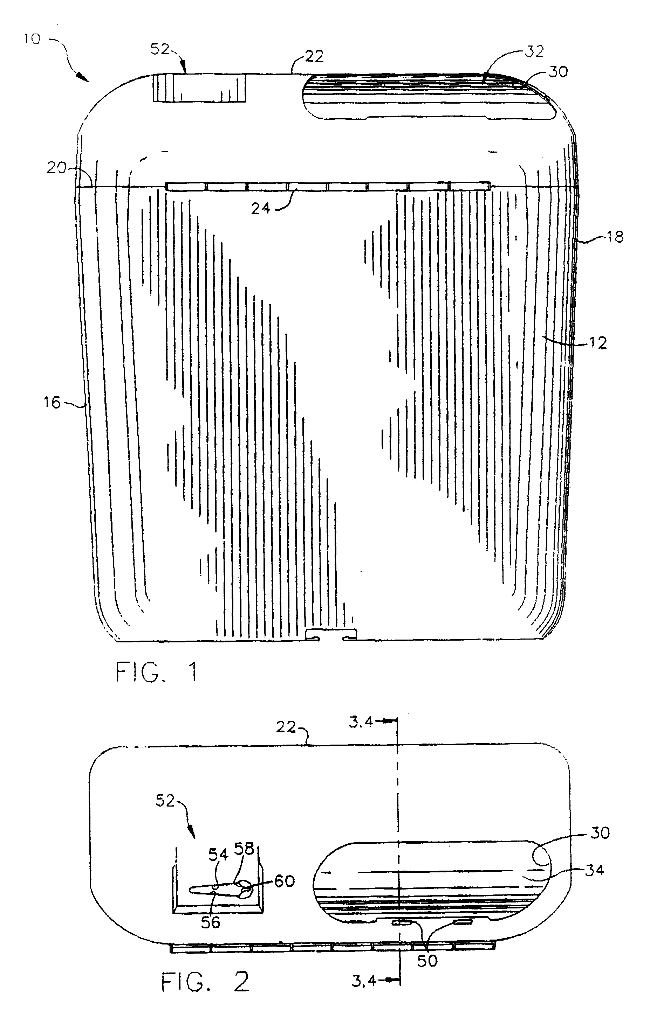

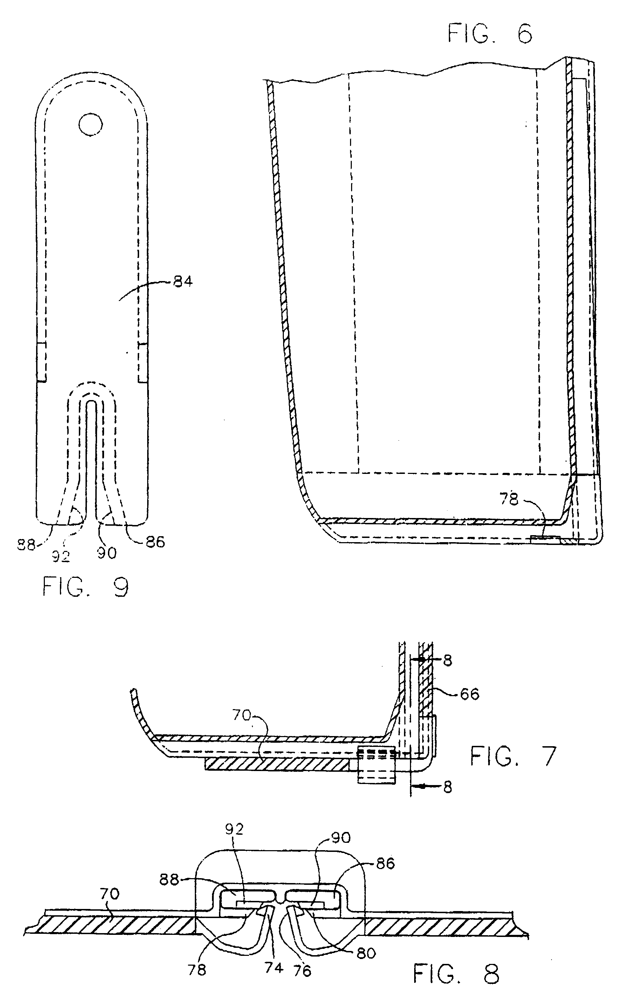

Referring to Fig. 1 of the drawing, there is illustrated an exemplary embodiment of the invention, which comprises a generally rectangular box-like housing, designated generally by the numeral 10, and formed of the usual plastic material for such containers.. The housing in its preferred form is formed of a lower substantially rectangular shell, having a front wall or panel 12 and a back wall or panel 14 (Fig. 5) defining front and back walls, and further including opposed side walls 16 and 18, all terminating at a top rectangular peripheral edge 20. A generally semi-circular top shell 22 is hinged at 24 to the top front edge of the rectangular shell 10.

The top 22 and bottom may be molded together with a live hinge or they be molded separately and utilize a snap lock hinge 24, as generally illustrated. A live hinge is a thin strip of the housing material connecting the two members and allowing them to pivot relative to one another. The hinge comprises a plurality of upwardly directed hooks on one member for hooking under a plurality of hinge pins formed or mounted in a plurality of tabs on the other member. The hooks have detents into which the hinge pin or pins snap into. A tab lock assembly includes tabs 26 on the top and tab sockets on the upper or top back edge of the container to permanently lock the top 22 in the covering position as shown.

The container top and bottom is preferably blow molded of a suitable plastic, and preferably formed with a slight taper from top to bottom to enable stacking for ease of shipment. The top 22 has a generally semi-cylindrical configuration about a horizontal axis, about which a pivoting closure member is mounted. Referring to Fig. 2, the top 22 is provided with a top front opening 30 of a generally elongated configuration having circular or oval ends. This opening is formed in the upper front one side of the top extending approximately one-half the length of the top. This opening is designed to receive syringes and the like for disposal and to secure the syringes against unauthorized retrieval.

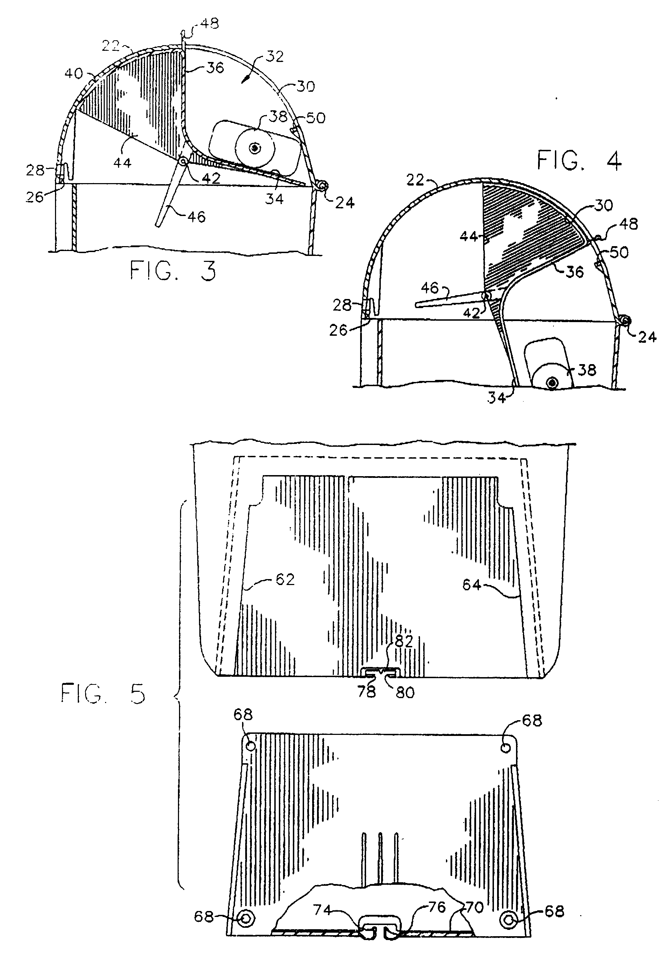

The opening is closed by a pivoting closure member or unit, as illustrated in Figs. 3-4, of a substantially unitary structure designated generally by the numeral 32. The closure member has a generally horizontal face or ledge portion 34 disposed below the opening 30, and a substantially vertical face portion 36 forming a continuation of the face 34, extending upward at approximately a one-hundred five degree angle. These faces together form a combination closure and receiving support for disposable articles, such as a syringe or the like 38 as illustrated. The closure unit 32 includes a curved surface portion 40 extending backward from the upper or top edge of the face portion 36, thereby forming a closure for extending over the opening 30 when the closure member pivots about horizontal axis 42 for dumping article 38. An end wall portion 44 of the closure member adds strength and rigidity to the closure unit.

The closure unit 32 is mounted for pivotal movement about a pair of pins, one at each end, which are rotatably mounted in snap in journals in the ends of the top. A counter weight 46 formed as an extending piece of the closure unit 32 extends downward from the axis of the pivot pins and pivot axis of the unit, and biases the closure unit to its normally closed article receiving position. Once the container has been filled, the closure is forced or rotated to its closed position, such that curved closure portion 40 extends over the opening 30, and lock tabs 48 on an upper portion of the closure panel 36 extend into and lock into lock slots or sockets 50, formed in a small horizontal lip at the forward edge of the housing opening. This essentially permanently locks the container in its closed position for disposal.

In normal operation, to dispose of a syringe as viewed in Fig. 2, the syringe is inserted into the top opening, with the needle extending toward the left to extend inside and underneath the top of the housing, such that the body of the syringe is placed on the horizontal closure surface 34, as seen in Fig. 3. The weight of the syringe overcomes the counterweight and tilts the closure member to dump the syringe into the container, as illustrated at Fig. 4. At the same time the closure surface 40 moves forward and covers the opening 30 to prevent the insertion of a hand or the like for the removal of articles from the container.

If it is desirable to remove the needle to either reuse the syringe or prior to disposing of the syringe, a needle removal slot, designed generally by the numeral 52, is formed in the upper portion of the top for this purpose. This needle removal slot has a generally tear-drop configuration converging from a larger diameter at one end to a smaller diameter at the other. The slot has a pair of opposed tapered side walls 54 and 56, which act as wrench surfaces for engaging the side of a needle hub for applying torque for unthreading the needle. A pair of opposed fingernail like edges 58 and 60 are provided at the larger end thereof for engaging between the threaded end of the shank of the needle and the syringe body to enable it to be pulled out of the syringe body.

Referring to Figs. 5-8, the container is designed for wall mounting by means of a lockable bracket that is preferably permanently attached to a wall or suitable wall like structure. The back of the container, as shown in Figs. 5 and 6, is provided with a recess having overlapped side portions 62 and 64 that receive a vertically extending back panel 66 secured to a wall or like vertical surface by means of suitable screws or the like in a plurality of holes 68. The bracket 66 includes a forwardly extending support shelf or surface 70, which is formed with a pair of lock hooks 74 and 76 at the center and at a position closely adjacent the back panel portion. These finger hooks or locks are designed to receive and latch to latching fingers 78 and 80 formed at the back end of a channel 82 formed along the bottom of the container.

The channel 82 is designed to receive latch releasing or unlocking key, as illustrated in Figs. 8 and 9, which comprises a main body member 84 having a pair of fingers 86 and 88 at a forward end thereof. The fingers are provided with camming surfaces 90 and 92 that extend downward for engaging and camming the lock hooks or fingers 74 and 76 out of latching engagement with latching fingers 78 and 80, as shown in Fig. 9. Thus, the container can be locked to the support bracket 66 and when ready for disposal or removal, a key 84 is selected and inserted in the slot 82 for engaging and releasing the lock or latch. The container can then be removed from the bracket and disposed of, and a new container mounted.

While I have illustrated and described my invention by means of specific embodiments, it is to be understood that numerous changes and modifications may be made therein without departing from the spirit and scope of the invention as defined in the appended claims.

## Claims

1. A secure disposable container assembly having a closure member for disposal of hospital sharps, comprising:
a generally box-like enclosed housing (10) having a top cover (22);
an access opening (30) in said top cover for receiving a disposable article; and
a closure member (32) for said access opening pivotally mounted within said top cover (22) for pivoting about a generally horizontal axis (42) having a front side and a back side, the closure member having a receptacle area disposed and extending radially outward from proximate said axis to a wall of said top cover on said front side of said axis, wherein said receptacle area is normally exposed to and covering said access opening (30) in a normally closed position for receiving a disposable article;
characterised in that:
the top cover (22) comprises a portion curving about said generally horizontal axis;
and
the closure member (32) comprises a self-dumping closure pivotally mounted for pivoting about said generally horizontal axis between a normally closed position and a dump position, means (46) for biasing said self-dumping closure to said normally closed position, said self-dumping closure having support means (34,36) defining said receptacle area and a curved surface (40), said means (36) comprising a surface extending upwardly from proximate said axis to said curved surface disposed above said generally horizontal axis for diverting said article to said receptacle area, said closure pivoting in response to said article on said receptacle area to said dump position for automatically dumping said article, and said curved surface (40) is moving to and covering said access opening upon pivoting of said self-dumping closure (32) from said normally closed position to said dump position.

2. A secure disposable container assembly according to claim 1 wherein:
said access opening (30) is an elongated horizontally extending slot.

3. A secure disposable container assembly according to claim 2 wherein:
said access opening (30) is positioned at one side of said top (22) and extends to proximate the center thereof.

4. A secure disposable container assembly according to claim 1 wherein:
said closure (32) comprises an elongated member extending the length of said top cover (22), and wherein said receptacle area is defined by a trough portion and said curved portion (40) is defined by an adjacent semi-cylindrical portion extending along above and on the opposite side of the axis (42) thereof.

5. A secure disposable container assembly according to claim 1 wherein:
said housing comprise a substantially rigid bottom shell defined by upstanding substantially rectangular walls (12,14,16,18) terminating in a generally rectangular rim (20) defining an upwardly extending top opening, said semi-cylindrical top is defined by a substantially semi-cylindrical top shell (22) hinged along an edge to an edge of said rim for covering said opening, and having locking tabs (26) for locking said top shell in position covering said top opening.

6. A secure disposable container assembly according to claim 5 wherein:
said housing having a back wall (14) having mounting means for mounting on a vertical support means, and a bottom having a latching slot (82) for engagement by a mount latch, and slide means (84) engaging a bottom of said housing for releasing said latch.

7. A secure disposable container assembly according to claim 1 wherein:
said pivotable closure includes locking means for permanently locking said closure in said second position.

8. A secure disposable container assembly according to claim 7 wherein:
said locking means comprises tabs (26) on said closure for engaging slots in said housing at a lower edge of said access opening (30).

9. A secure disposable container assembly according to claim 8 wherein:
said closure member comprises an elongated member extending the length of said top cover, and wherein said receptacle area is defined by a trough portion and said curved portion (40) is defined by an adjacent semi-cylindrical portion extending along the axis thereto.

10. A secure disposable container assembly according to claim 7 wherein:
said generally box-like enclosed housing (10) comprises a substantially rigid bottom shell defined by upstanding substantially rectangular walls (12,14,16,18) terminating in a generally rectangular rim (20) defining an upwardly extending top opening, said top cover defined by a substantially semi-cylindrical top shell (22) curving about said generally horizontal axis (42) hinged along an edge to an edge of said rim for covering said opening, locking tabs (26) for locking said top shell in position covering said top opening;
said access opening comprising an elongated horizontally extending opening (30) in said top shell.

## Patentansprüche

1. Eine sichere Wegwerfbehälterbaugruppe mit einem Verschlußelement zur Entsorgung von scharfen Krankenhausgegenständen,
die ein allgemein kastenförmiges, in sich geschlossenes Gehäuse (10) mit einer oberen Abdeckung (22) umfaßt;
eine Zugangsöffnung (30) in der oberen Abdeckung zum Empfangen eines Wegwerfartikels umfaßt; und
für die Zugangsöffnung eine zur Drehung um eine allgemein horizontale Achse (42) drehbar in der oberem Abdeckung (22) angebrachtes Verschlußelement (32) mit einer Vorderseite und einer Hinterseite umfaßt, wobei das Verschlußelement eine Empfangsfläche bereitstellt und sich von der Nähe der Achse radial nach außen zu einer Wand der oberen Abdeckung auf der Vorderseite der Achse erstreckt, wobei die Empfangsfläche normalerweise an der Zugangsöffnung (30) exponiert liegt und diese in einer normalerweise geschlossenen Position zum Empfang eines Wegwerfartikels abdeckt;
**dadurch gekennzeichnet,**
daß die obere Abdeckung (22) einen sich um die allgemein horizontale Achse krümmenden Abschnitt umfaßt;
und das Verschlußelement (32)
einen um die generell horizontale Achse zwischen einer normalerweise geschlossenen Position und einer Abladeposition drehpunktgelagerten, selbstabladenden Verschluß umfaßt und
eine Einrichtung (46) zur Vorspannung des selbstabladenden Verschlusses in die normalerweise geschlossene Position, wobei der selbstabladende Verschluß eine die Empfangsfläche bildende Stützeinrichtung (34, 36) und eine gekrümmte Oberfläche (40) besitzt, wobei die Einrichtung (36) eine sich nahe der Achse nach oben zu der gekrümmten Fläche erstreckende Oberfläche umfaßt, die über der allgemein horizontalen Achse zur Ableitung des Gegenstandes in die Empfangsfläche angeordnet ist, wobei sich der Verschluß aufgrund des auf der Empfangsfläche befindlichen Gegenstands zur automatischen Abladung des Gegenstands in die Abladeposition dreht und sich die gekrümmte Oberfläche (40) bei Drehung des selbstabladenden Verschlusses (32) aus seiner normalerweise geschlossenen Position in die Abladeposition auf die Zugangsöffnung zu bewegt und diese abdeckt.

2. Eine sichere Wegwerfbehälterbaugruppe nach Anspruch 1, worin die Zugangsöffnung (30) ein länglicher, sich horizontal erstreckender Schlitz ist.

3. Eine sichere Wegwerfbehälterbaugruppe nach Anspruch 2, worin die Zugangsöffnung (30) auf einer Seite des Oberteils (22) liegt und sich bis in die Nähe dessen Mitte erstreckt.

4. Eine sichere Wegwerfbehälterbaugruppe nach Anspruch 1, worin der Verschluß (32) ein sich entlang der Länge der oberen Abdeckung (22) erstreckendes, längliches Element unfaßt, und worin die Empfangsfläche durch einen Trogteil definiert ist und der gekrümmte Abschnitt (40) durch einen angrenzenden, sich daran entlang über und auf der gegenüberliegenden Seite der Achse (42) erstreckenden, halbzylindrischen Abschnitt definiert ist.

5. Eine sichere Wegwerfbehälterbaugruppe nach Anspruch 1, worin das Gehäuse eine im wesentlichen steife Bodenschale umfaßt, die durch aufrechtstehende, im wesentlichen rechtwinklige Wände (12, 14, 16, 18) definiert ist, welche in einem allgemein rechtwinkligen, eine aufwärts gerichtete, obere Öffnung definierenden Rand (20) enden und das halbzylindrische Oberteil durch eine im wesentlichen halbzylindrische obere Schale (22) definiert ist, welche zur Bedeckung der Öffnung entlang einer Kante scharnierartig mit einer Kante des Randes verbunden ist und Verschlußaufhänger (26) zum Verschließen der oberen Schale in einer die obere Öffnung abdeckenden Position besitzt.

6. Eine sichere Wegwerfbehälterbaugruppe nach Anspruch 5, worin das Gehäuse eine Hinterwand (14) mit Befestigungsmitteln zur Befestigung an einer vertikalen Stützeinrichtung besitzt, und einer Boden mit einem Verriegelungsschlitz (82) zum Eingriff mit einem Befestigungsriegel, und eine Gleiteinrichtung (84), die in einen Boden des Gehäuses zum Lösen des Riegels eingreift.

7. Eine sichere Wegwerfbehälterbaugruppe nach Anspruch 1, worin der drehbar gelagerte Verschluß eine Schließeinrichtung zum permanenten Verschließen des Verschlusses in der zweiten Position enthält.

8. Eine sichere Wegwerfbehälterbaugruppe nach Anspruch 7, worin die Verschlußeinrichtung Aufhänger (26) an dem Verschluß für den Eingriff in Schlitzen im Gehäuse an einer unteren Kanten der Zugangsöffnung (30) umfaßt.

9. Eine sichere Wegwerfbehälterbaugruppe nach Anspruch 8, worin das Verschlußelement ein sich entlang der Länge der oberen Abdeckung erstreckendes, längliches Element umfaßt, und worin die Empfangsfläche durch einen Trogabschnitt definiert ist, und der gekrümmte Abschnitt (40) durch einen angrenzenden halbzylindrischen Abschnitt definiert ist, der sich entlang der Achse dazu ausdehnt.

10. Eine sichere Wegwerfbehälterbaugruppe nach Anspruch 7, worin das allgemein kastenförmige, in sich geschlossene Gehäuse (10)
eine im wesentlichen steife Bodenschale, die durch aufrechtstehende, in wesentlichen rechtwinklige Wände (12, 14, 16, 18), welche in einem allgemein rechtwinkligen, eine aufwärts gerichtete, obere Öffnung definierenden Rand (20) enden, definiert ist, wobei die obere Abdeckung durch eine im wesentlichen halbzylindrische, sich über die allgemein horizontale Achse (42) krümmende obere Schale (22), welche zur Abdeckung der Öffnung entlang einer Kante scharnierartig mit einer Kante des Randes verbunden ist, definiert ist,
Verschlußaufhänger (26) zum Verschließen der oberen Schale in einer die obere Öffnung abdeckenden Position umfaßt; und
die Zugangsöffnung eine sich länglich, horizontal erstreckende Öffnung (30) in der oberen Schale umfaßt.

## Revendications

1. Ensemble récipient jetable de sûreté comportant un élément de fermeture pour la mise au rebut d'objets tranchants hospitaliers, comprenant :
une enceinte close généralement en forme de boite (10) comportant un couvercle supérieur (22) ;
un orifice d'accès (30) dans ledit couvercle supérieur pour recevoir un article à jeter ; et
un élément de fermeture (32) pour ledit orifice d'accès monté de façon pivotante a l'intérieur dudit couvercle supérieur (22) pour pivoter autour d'un axe généralement horizontal (42) ayant une face avant et une face arrière, l'élément de fermeture comportant une zone de réception située et s'étendent de façon radiale vers l'extérieur partant immédiatement dudit axe vers une paroi dudit couvercle supérieur sur ladite face avant dudit axe, dans lequel ladite zone de réception est normalement exposée audit orifice d'accés (30) et le recouvre dans une position normalement fermée pour recevoir un article à jeter ; caractérisé en ce que :
le couvercle supérieur (22) comprend une partie incurvée autour dudit axe généralement horizontal;
et l'élément de fermeture (32) comprend une fermeture auto-basculante montée de façon pivotante pour pivoter autour dudit axe généralement horizontal entre une position normalement fermée et une position de basculement, des moyens (46) pour dévier ladite fermeture auto-basculante à ladite position normalement fermée, ladite fermeture auto-basculante comportant des moyens formant support (34, 36) définissant ladite zone de réception, et une surface incurvée (40), lesdits moyens (36) comprenant une surface s'étendant vers le haut depuis immédiatement ledit axe vers ladite surface incurvée disposée au-dessus dudit axe généralement horizontal pour dévier ledit article vers la zone de réception, ladite fermeture pivotant en réaction audit article dans ladite zone de réception à ladite position de basculement pour faire basculer automatiquement ledit article, et ladite surface incurvée (40) se déplace sur et recouvre ledit orifice d'accès par pivotement de ladite fermeture auto-basculante (32) depuis ladite position normalement fermée à ladite position de basculement.

2. Ensemble récipient jetable de sûreté selon la revendication 1 dans lequel :
ledit orifice d'accès (30) est une fente allongée s'étendant horizontalement.

3. Ensemble récipient jetable de sûreté selon la revendication 2 dans lequel :
ledit orifice d'accès (30) est positionné sur un côté dudit couvercle supérieur (22) et s'étend jusqu'à proximité du centre de celui-ci.

4. Ensemble récipient jetable de sûreté selon la revendication 1 dans lequel :
ladite fermeture (32) comprend un élément allongé s'étendant sur la longueur dudit couvercle supérieur (22), et dans lequel ladite zone de réception est définie par une partie en creux et ladite partie incurvée (40) est définie par une partie adjacente semi-cylindrique s'étendant au-dessus et sur le côté opposé de l'axe (42) de celle-ci.

5. Ensemble récipient jetable de sûreté selon la revendication 1 dans lequel :
ladite enceinte comprend une enveloppe extérieure inférieure sensiblement rigide définie par des parois verticales sensiblement rectangulaires (12, 14, 16, 18) se terminant par un rebord généralement rectangulaire (20) définissant un orifice supérieur s'étendant vers le haut, ledit couvercle supérieur semi-cylindrique est défini par une enveloppe extérieure supérieure sensiblement semi-cylindrique (22) fixée par charnière sur un bord à un bord dudit rebord pour recouvrir ledit orifice, et comportant des pattes de fermeture (26) pour fermer ladite enveloppe extérieure supérieure en position de recouvrement dudit orifice supérieur.

6. Ensemble récipient jetable de sûreté selon la revendication 5 dans lequel :
ladite enceinte comportant une paroi arrière (14) ayant des moyens de montage pour être montée sur des moyens formant support vertical, et un fond comportant une encoche de fixation (82) pour engagement sur un loquet de montage, et des moyens de glissement (84) engageant un fond de ladite enceinte pour débloquer ladite fixation.

7. Ensemble récipient jetable de sûreté selon la revendication 1 dans lequel :
ladite fermeture pivotante comprend des moyens de fermeture pour fermer en permanence ladite fermeture dans ladite seconde position.

8. Ensemble récipient jetable de sûreté selon la revendication 7 dans lequel :
lesdits moyens de fermeture comprennent des pattes (26) sur ladite fermeture pour s'engager dans des encoches dans ladite enceinte sur le bord inférieur dudit orifice d'accès (30).

9. Ensemble récipient jetable de sûreté selon la revendication 8 dans lequel :
ledit élément de fermeture comprend un élément allongé s'étendant sur la longueur dudit couvercle supérieur, et dans lequel ladite zone de réception est définie par une partie en creux et ladite partie incurvée (40) est définie par une partie adjacente semi-cylindrique s'étendant le long de l'axe de celle-ci.

10. Ensemble récipient jetable de sûreté selon la revendication 7 dans lequel :
ladite enceinte close généralement en forme de boite (10) comprend une enveloppe extérieure inférieure sensiblement rigide définie par des parois verticales sensiblement rectangulaires (12, 14, 16, 18) se terminant par un rebord généralement rectangulaire (20) définissant un orifice supérieur s'étendant vers le haut, ledit couvercle supérieur défini par une enveloppe extérieure supérieure sensiblement semi-cylindrique (22) s'incurvant autour dudit axe généralement horizontal (42) fixée par charnière sur un bord à un bord dudit rebord pour recouvrir ladite ouverture, des pattes de fermeture (26) pour fermer ladite enveloppe extérieure supérieure en position de recouvrement dudit orifice supérieur ;
ledit orifice d'accès comprenant une ouverture allongée s'étendant horizontalement (30) dans ladite enveloppe extérieure supérieure.
